# EUROPEAN PATENT SPECIFICATION

(11) **EP 1 667 605 B1**
(45) Date of publication and mention of the grant of the patent: **17.12.2008**
(21) Application number: 04782617.7
(22) Date of filing: 30.08.2004
(51) Int. Cl.: A61F 2/90

(54) **RADIOPAQUE MARKERS FOR MEDICAL DEVICES**
RÖNTGENDICHTE MARKER FÜR MEDIZINPRODUKTE
MARQUEURS RADIO-OPAQUES POUR DISPOSITIFS MEDICAUX

(30) Priority: 12.09.2003 US 661361
(43) Date of publication of application: 14.06.2006
(73) Proprietor: GUIDANT ENDOVASCULAR SOLUTIONS, Santa Clara, CA 95054 (US)
(72) Inventor: MACKIEWICZ, David, A., Scotts Valley, CA 95066 (US); FITZGERALD, Keif, San Jose, CA 95132 (US); ANUKHIN, Boris, San Jose, CA 95129 (US)
(74) Representative: McLeish, Nicholas Alistair Maxwell
(86) International application number: PCT/US2004/028179
(87) International publication number: WO 2005/032403

(56) References cited:
- WO-A-20/04016199
- WO-A-20/04060213
- WO-A-20/04103217
- WO-A-20/04105642
- US-A- 5 741 327
- US-B1- 6 334 871
- US-B2- 6 503 271

## Description

### BACKGROUND OF THE INVENTION

The present invention generally relates to implantable medical devices, such as endoprosthesic devices generally known as stents, and more particularly, to radiopaque markers which can be used with such medical devices to increase the visualization of the implanted medical device during fluoroscopy or by x-ray.

Stents are typically implanted in a body lumen, such as carotid arteries, coronary arteries, peripheral arteries, veins, or other vessels to maintain the patency of the lumen. These devices are frequently used in the treatment of atherosclerotic stenosis in blood vessels especially after percutaneous transluminal angioplasty (PTA) or percutaneous transluminal coronary angioplasty (PTCA) procedures with the intent to reduce the likelihood of restenosis of a vessel. Stents also are used to support a body lumen, tack-up a flap or dissection in a vessel, or in general where the lumen is weak to add support. Stents, or stent-like devices, are often used as the support and mounting structures for implantable vascular grafts which can be used to create an artificial conduit to bypass a diseased portion of the vasculature, such as an abdominal aortic aneurysm.

Expandable stents can be delivered on expandable catheters, such as balloon catheters, in which the stent is positioned over the balloon portion of the catheter and expanded from a reduced diameter to an enlarged diameter through inflation of the balloon. Such expandable stents are often made from stainless steel alloys, such as Stainless Steel 316L or cobalt-chromium alloys, which may provide the necessary scaffolding properties needed to support the body lumen, but may not possess an appreciable level of radiopacity to allow the stent to be highly visible during fluoroscopy or by x-ray. Radiopacity permits the cardiologist or physician to visualize the procedure involving the stent through the use of fluoroscopes or similar radiological equipment. Visualization of the implanted stent is also important during subsequent checkups in which the positioning of the stent in the body vessel can be monitored.

Self-expanding stents, as the name implies, are stents which self-expand through the properties of the material constituting the stent. Inflation force developed by a balloon catheter is usually not necessary to deploy this kind of stent. Self-expanding stents are highly resilient and springy which allows the stent to spring back to a desired shape even after being squeezed or crushed. Important applications for self-expanding stents include implantation at body locations, such as the neck or in peripheral arteries and veins, where the stent may be located close to a surface of the body which is particularly vulnerable to impact forces that can partially or completely collapse the stent and cause similar partial or total vessel blockage. In such body locations, a balloon expandable stent may not be the best scaffolding device since such a stent can remain crushed if subjected to an unwanted force. These important applications have prompted designers to seek out superelastic and shape memory alloys to exploit the materials' properties in their self-expanding stents. Nickel-titanium is an often used alloy for such medical applications. Clearly, self-expanding, nickel-titanium stents are useful and valuable to the medical field. However, a distinct disadvantage with self-expanding, nickel-titanium made stents is the fact that they have an even lower level of radiopacity than balloon expandable stents made from materials such as stainless steel or cobalt-chromium alloy. Good radiopacity is therefore a useful feature for both conventional balloon-expandable stents and self-expanding nickel-titanium stents to possess.

Radiopacity of the stent can be somewhat improved by increasing the strut thickness of the stent. However, increasing the strut thickness usually detrimentally affects the flexibility of the stent, which is a quality necessary for ease of delivery through the patient's vasculature. A stent having increased strut thickness also can be more difficult to deploy from the catheter delivery system. Another complication is that radiopacity and radial force co-vary with strut thickness. Also, nickel-titanium is somewhat difficult to machine and thick struts can exacerbate the problem.

Radiopacity can be improved through coating processes such as sputtering, plating, or co-drawing gold or similar metals onto the stent. These processes, however, can create complications such as material compatibility, galvanic corrosion, high manufacturing cost, coating adhesion or delamination, biocompatibility, loss of coating integrity following collapse and deployment of the stent, etc. Coatings also can affect the mechanical properties of the stent, such as flexibility and the amount of radial force that can be attained and delivered to the body vessel.

Radiopacity can be improved by using radiopaque markers which can be attached to the struts forming the stent. In this manner, materials which have higher radiopacity than the stent structure itself, such as gold, tantalum or platinum, have been utilized as markers and have been strategically placed along the body of the stent to increase the visualization of the stent.

For example, US-A-5,741,327 describes radiopaque marker elements for attachment to ends of a radially expandable surgical stent. Each radiopaque marker element is homogeneously formed from a radiopaque material and attached through an attachment means to ends of the stent. The marker elements extend beyond ends of the stent. The marker elements are attached to the stent before radial expansion and are configured to radially expand along with the stent during surgical implantation thereof within a body lumen, such as an artery. The radiopaque marker elements can either be attached to an unmodified radially expandable surgical stent or to a prepped stent which includes receivers at ends of the stent particularly configured to attach to the radiopaque marker elements. The radiopaque marker elements include tabs which match a contour of the receivers so that secure attachment of the radiopaque marker elements to the receivers at the ends of the stent is provided.

By contrast, US-A-6,334,871 describes stents having radiopaque markers in the form of rivets made of a material which is more radiopaque than the stent substance so the location of the stent can be identified. Preferably the stents are heat treated so that atoms from the stent material migrate into the marker material and vice versa.

WO 2004/105642 describes a connection system for connecting a stent to a radiopaque marker. The connection system supposedly permits a connection of the stent to a radiopaque marker without a reduction of the mechanical properties of the stent and provides an adequate retaining force for the probing and implantation of the stent. The connection system comprises at least one tensioning connection of a tensioning element and clamping element.

However, there are problems sometimes associated with the implementation and attachment of such radiopaque markers to the stent structure. Such complications include difficulty in welding or bonding the marker to the stent structure due to the material incompatibility, galvanic corrosion which could be caused through the use of different materials for the markers and the stent, along with higher manufacturing costs due to the often labor intensive techniques required in order to properly attach the markers to the stent. Additionally, the markers must be attached to the stent structure so as not to affect the mechanical properties (i.e. radial force, flexibility, etc.) of the stent, both during delivery and during deployment.

What has been needed is an improved radiopaque marker system that increases the radiopacity of a stent, yet preserves the mechanical properties of the stent. Such a marker system must be easy to attach to the stent structure to provide increased radiopacity to the composite device, again, without adversely affecting the physical properties of the stent or causing unwanted galvanic corrosion. The present invention satisfies these and other needs.

### SUMMARY OF THE INVENTION

According to the present invention there is provided an implantable medical device having enhanced radiopacity, comprising: a structural body formed from a biocompatible material having a certain level of radiopacity, the structural body including at least one marker holder integrally formed therein; and a radiopaque marker made from a material having a level of radiopacity greater than the level of radiopacity of the biocompatible material from which the structural body is formed, the radiopaque marker being attachable within the marker holder, wherein the marker holder includes a pair of projecting fingers which define an opening and the radiopaque marker includes a region which fits within the opening defined by the projecting fingers,
**characterised in that** the opening defined by the projecting fingers of the marker holder is substantially V-shaped and the region formed on the radiopaque marker to fit within the opening is substantially V-shaped.

Thus the present invention relates to an implantable medical device, such as a stent, for use or implantation in the body or a body lumen. In one aspect of the present invention, the implanted medical device includes a structural body made from a superelastic material, such as a nickel-titanium alloy, which attains a certain level of radiopacity. The structural body includes one or more marker holders which are integrally formed with the structural body. Each marker holder is designed to hold a radiopaque marker which has a level of radiopacity greater than the superelastic material. In one aspect, the radiopaque marker can be made from a nickel-titanium alloy which includes a ternary element. A ternary element is selected from a group of materials having a high level of radiopacity. For example, the ternary element can be selected from the group of elements consisting of iridium, platinum, gold, rhenium, tungsten, palladium, rhodium, tantalum, silver, ruthenium, and hafnium. In one particular embodiment of the present invention, the radiopaque marker is made from an alloy having 42.8 atomic percent nickel, 49.7 atomic percent titanium, and 7.5 atomic percent platinum. Such an alloy possesses sufficient radiopacity to create a marker system which enhances the visualization of the composite medical device during fluoroscopy or by x-ray, without affecting the physical properties of the device. Also, possible galvanic corrosion between the different alloys is eliminated altogether, or at least greatly reduced.

The medical device includes a structural body having one or more marker holders adapted to receive a radiopaque marker. Each marker holder may include a pair of projecting fingers connected together at a notched region to cooperatively create a V-shaped opening. This V-shaped opening, in turn, is adapted to receive a portion of the radiopaque marker which fits within the opening. The portion of the radiopaque marker is V-shaped as well. The V-shaped opening formed by the pair of projecting fingers creates a mounting region that allows the projecting fingers to move outwardly, if necessary, in order to receive the V-shaped portion of the radiopaque marker. In this regard, such a mounting structure allows the marker holder to easily compensate for derivations caused by an imprecise fit between the radiopaque maker and the pair of projecting fingers. Melting or heat welding at the abutment of the radiopaque marker with the projecting fingers securely affixes the components together. Such a marker system can be implemented on a number of implantable medical devices, including self-expanding stents and balloon expandable stents.

In arrangements falling outside the scope of the present invention, the marker holder can take on a different configuration from the embodiment briefly described above. In one particular arrangement the marker holder is created with a rectangular opening, which is adapted to receive the radiopaque marker having a comparable size and shape. In this arrangement the radiopaque marker includes an inner core which is partially, or completely, encased by an outer layer. This inner core can be made from a highly radiopaque material, such as palladium, gold, or the like. The outer layer, in turn, can be formed from a material which may be easier to weld to the marker holder and may be more compatible with the marker holder to prevent galvanic corrosion. In this arrangement the inner core material does not contact the marker holder so material incompatibility which may affect the ability to weld the component or promote galvanic corrosion is minimized. In one arrangement the outer layer can be made with the same material used to form the marker holder. Melting or heat welding is utilized to melt a portion of the marker holder and the outer layer to meld the components together.

In still another arrangement the marker holder can be made from a self-expanding material which utilizes a phenomenon referred to as the shape memory effect (SME) to lock the radiopaque marker in place. A shape-memory alloy (SMA) utilizes unique properties which allow the material to assume different shapes at different temperatures. Nickel-titanium alloy is a suitable SMA which could be utilized to create a marker holder which assumes a particular shape at one temperature and another shape at a different temperature. In this regard, the marker holder can be designed with an opening having a shape which normally would not be sufficiently large enough to receive the radiopaque marker, but could be deformed to another shape which readily accepts the particular size and shape of the radiopaque marker. This mounting of the marker in the opening can be performed by subjecting the marker holder to a different temperature to obtain the alternate shape. Such a marker holder could then be brought back to the first temperature which forces the marker holder into the alternative configuration, causing the marker holder to tightly grasp the marker, virtually locking it in place. The marker holder and radiopaque markers which utilize the shape memory effect as a mounting system can be adapted to a variety of shapes and sizes. Alternatively, the superelastic property of nickel-titanium also could be utilized in a similar fashion to create a similar locking system.

Other features and advantages of the present invention will become more apparent from the following detailed description of the invention when taken in conjunction with the accompanying exemplary drawings.

### BRIEF DESCRIPTION OF THE DRAWINGS

FIGURE 1 is a plan view of a flattened strut pattern of an exemplary stent having radiopaque markers and marker holders made in accordance with the present invention attached to the outermost ends of the stent.
FIG. 2 is an elevational view of an embodiment of a radiopaque marker attached to a marker holder formed on the stent structure depicted in FIG. 1.
FIG. 3 is an elevational view showing the particular shape of the radiopaque marker and the V-shaped opening of the marker holder depicted in FIG. 2.
FIG. 4 is an elevational view of another radiopaque marker attached to a marker holder formed on a stent strut.
FIG. 5 is a perspective view of a radiopaque marker having a core encapsulated by an outer layer which can be placed in either of the marker holders depicted in FIG. 4 and FIGS. 6 and 7.
FIG. 6 is an alternative marker holder which can be formed on a nickel-titanium stent having shape memory effect prior to insertion of the radiopaque marker.
FIG. 7 is an elevational view of the marker holder of FIG. 6 including a radiopaque marker, such as the one depicted in FIG. 5, inserted and securely attached to the marker holder.

### DETAILED DESCRIPTION OF THE PREFERRED EMBODIMENTS

The present invention relates to radiopaque markers used to enhance the radiopacity of an implantable medical device. For the sake of illustration, the following exemplary embodiments are directed to stents, although it is understood that the present invention is applicable to other medical devices which are implantable in a body lumen as well as other parts of the body.

The stent embodiment of the present invention can have virtually any configuration that is compatible with the body lumen in which they are implanted. The stent should preferably be configured so that there is a substantial amount of open area. The stent should also be configured so that dissections or flaps in the body lumen wall are covered and tacked up by the stent.

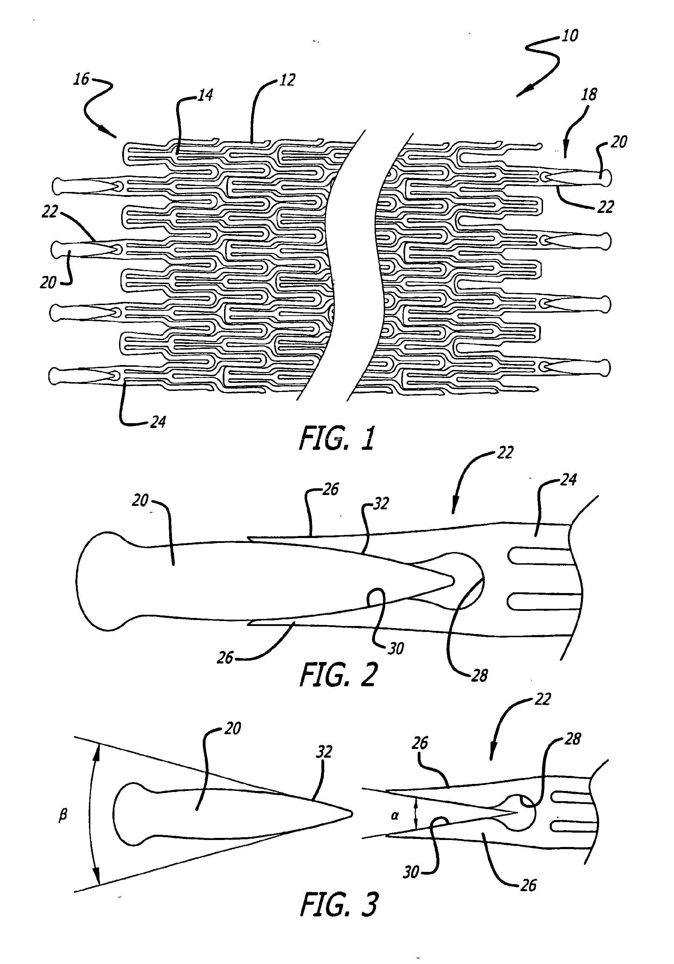

Referring now to FIG. 1, in one particular embodiment of the present invention, a stent 10 is formed partially or completely from an alloy such as nitinol (NiTi), which has superelastic (SE) characteristics. The strut pattern of stent 10 is somewhat similar to the strut pattern disclosed in United States Patent No, 6,537,311, "Stent Designs for Use in Peripheral Vessels" issued to Cox et al. on March 25, 2003.

Of course, the configuration of the stent 10 is just one example of many stent configurations that are contemplated by the present invention.

In use, the stent 10 takes on a tubular form (not shown) which preferably includes a plurality of radially expandable cylindrical elements 12 disposed generally coaxially and interconnected by members 14 disposed between adjacent cylindrical elements 12. The shapes of the struts forming the strut pattern are designed so they can preferably be nested. This strut pattern is best seen from the flattened plan view of FIG. 1. The serpentine patterned struts are nested such that the extended portions of the struts of one cylindrical element 12 intrude into a complementary space within the circumference of an adjacent cylindrical element. In this manner, the plurality of cylindrical elements 12 can be more tightly packed lengthwise.

In actual use, the stent 10 forms a structural body capable of expanding from a collapsed delivery position to an expanded position for implantation within, for example, an artery or vein of a patient. As can be seen in FIG. 1, the stent 10 has a first end 16 and a second end 18 which define the overall longitudinal length of the stent. It should be appreciated that the stent can be manufactured in a number of different lengths, depending upon the particular application and location in which the device will be implemented. Attached to the first end 16 and second end 18 are a number of radiopaque markers 20 which provide increased radiopacity to the composite device. These radiopaque markers 20 are designed from materials having a higher level of radiopacity than the material utilized to form the stent 10 to increase the visualization of the stent at least along the ends 16 and 18 of the stent. In this regard, the radiopaque markers provide the physician with visual set points during fluoroscopy or by x-ray to help the physician visualize the positioning of the stent in the body lumen. It should be appreciated that although the radiopaque markers 20 are shown located at the first and second ends of the stent 10, such radiopaque markers could also be placed at various locations along the length of the stent to provide the necessary visualization points for the physician.

Each of the radiopaque markers 20 are attached to the ends of the stent via marker holders 22 which extend from the cylindrical elements 12 formed at the opposing ends of the stent. As will be discussed in greater detail below, these marker holders 22 provide a mounting system which should provide the stent manufacturer with greater ease in attaching the markers 20 to the stent, or any other medical device which may benefit from increased radiopacity.

As can be best seen in FIGS. 2 and 3, the particular design of the marker holders 22 is shown extending from one of the W-shaped peaks 24 formed on the cylindrical element 12 of the stent 10. Each of the marker holders 22 include a pair of projecting fingers 26 connected at a notched region 28. The pair of projecting fingers 26 create a V-shape opening 30 which is adapted to receive a V-shaped end 32 of the radiopaque marker 20. As can best be seen in FIG. 3, the V-shaped opening 30 formed by the pair of projecting fingers 26 creates an angle α which can measure from about 10°-60°. The notched region 28 creates a spring-like mechanism by allowing the projecting fingers 26 to move inwardly or outwardly, if necessary, in order to receive the V-shaped end 32 of the radiopaque marker 20. The V-shaped end 32 of the radiopaque marker 20 creates an angle β which is substantially the same size as angle α, except that the angle β may be slightly larger in order to achieve a snug fit of the V-shaped end 32 into the V-shaped opening 30. Accordingly, when the V-shaped end 32 is placed in the V-shaped opening 30, the projecting fingers 26 can move outward, if necessary, in order to create a snug fit between these two components, as is shown in FIG. 2.

In order to assure that the radiopaque marker 20 remains securely attached to the marker holder 22, melting or heat welding is utilized to join the radiopaque marker and marker holder together. In this regard, the joint can be either a conventional weld, in which additional material is heated with the components to bond the components together, or the joint can be a heat weld in which heat from a source, such as a laser, melts a portion of the projecting fingers and the radiopaque marker to melt the components together. It should also be appreciated other ways of bonding or permanently affixing the radiopaque marker 20 onto the marker holder 22 can be utilized without departing from the scope of the present invention.

The radiopaque marker 20 and marker holder 22 shown in FIGS. 1-3 can be utilized in conjunction with a number of different medical devices including a self-expanding stent or a balloon-expandable stent to provide increased radiopacity to the device. The radiopaque marker 20 should be fabricated from a material having a level of radiopacity which is much greater than the level of radiopacity of the underlying material which forms the structure of the medical device. Also, while the marker holder 22 is shown as integrally formed as part of the stent pattern, it should also be appreciated that the marker holder 22 could be manufactured as a separate component that can be bonded or otherwise attached to a portion of an implantable medical device. However, the forming of the marker holders directly with the body structure increases the structural integrity of the medical device and eliminates the need to bond or otherwise attach the marker holder onto the medical device. This again simplifies the manufacturing process.

The projecting fingers 26 of the marker holder 22 provide a bonding surface away from high stress areas, as is shown in the embodiment of FIG. 1. In this regard, the fingers are placed along the strut pattern of the stent in such a fashion as to prevent or minimize any interference in the mechanical properties of the stent, i.e. the flexibility and the radial force developed by the stent during use. Additionally, the V-shaped angle of the opening reduces tolerance sensitivity which may be created once the stent is electropolished. In this regard, the ability of the projecting fingers to move relative to the V-shaped region of the radiopaque marker 10 compensates for any material loss that may be caused during the electropolishing of the components. The notched region 28 lends some degree of flexibility to the projecting fingers 26 to allow tight contact with the V-shaped edge 32 of the radiopaque marker 20.

In one particular form of the present invention, the stent 10 can be manufactured form a nickel-titanium alloy which has superelastic properties. The radiopaque marker 20 can be manufacture from a ternary nickel-titanium alloy, such as a nickel-titanium-platinum alloy. Such a ternary nickel-titanium alloy is disclosed in U.S. Patent No. 6,855,161 filed December 27, 2000.

Such a ternary alloy possesses a higher level of radiopacity than the nickel-titanium alloy utilized to fabricate the self-expanding stent. It should be appreciated that still other ternary nickel-titanium alloys could be utilized, i.e. ternary elements selected from the group including iridium, platinum, gold, rhenium, tungsten, palladium, rhodium, tantalum, silver, ruthenium, and hafnium and the like. Since the binary nickel-titanium alloy used to create the stent is highly compatible with a ternary nickel-titanium alloy, there is little possibility that the two components will cause any galvanic corrosion and does not affect the ability to properly join the two components together.

In an embodiment in which the underlying stent structure is made from a stainless steel material, such as Spring Steel 316L, or a cobalt chromium alloy, again, a number of different materials could be utilized to form the radiopaque marker to increase the over all level of radiopacity of the composite device. Those skilled in the art will recognize the number of possible materials that could be utilized as a suitable radiopaque material for use in such an embodiment of the present invention.

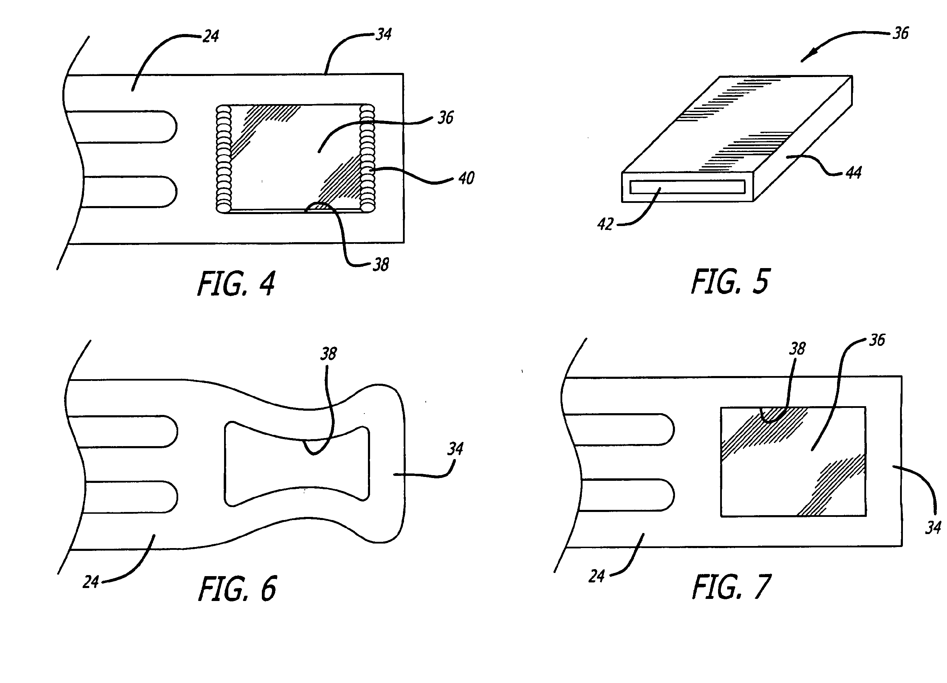

Referring now to FIGS. 4 and 5, another marker holder 34 and radiopaque marker 36 is shown. As can be seen from FIG. 4, the marker holder 34 is somewhat different from the marker holder of FIGS. 1-3 in that a somewhat rectangular shaped opening 38 is formed at the end of the stent structure 10 which is adapted to accept the marker 36. The radiopaque marker 36 also has a substantially rectangular shape as best can be seen in FIG. 5. In this particular arrangement the radiopaque marker 36 is designed to fit within the opening 38 of the marker holder 34. As can be seen in FIG. 4, the radiopaque marker 36 can be seemingly attached within this opening 38 via the use of welds 40 which are utilized to permanently affix the marker within the opening 38. In this manner, the radiopaque marker should remain securely affixed to the holder.

The particular radiopaque marker shown in FIG. 5 includes an inner core 42 which is encased by an outer layer 44. In an exemplary arrangement this inner core 42 is made from a material, such as palladium, gold, or the like, which has a high level of radiopacity. The outer layer 44, in turn, can be formed form a material which may be easier to weld to the marker holder 34 and, in one arrangement can be made from the same material which forms the stent and marker holder. In this arrangement the outer layer 44 can be made from a material such as a binary nickel-titanium alloy or a ternary nickel-titanium alloy which could be the same material utilized to form the stent structure and marker holder 34. In this regard, heat welds 40 can be utilized to melt a portion of the marker holder 34 and the outer layer 44 to properly attach the radiopaque marker into the marker holder. The inner core 42 can be made from a very highly radiopaque material, such as platinum and similar materials.

One of the benefits of using a highly radiopaque material at least partially encapsulated by an outer layer is the elimination of the need to directly weld the core material to the marker holder. In this regard, platinum, which may be somewhat difficult to weld to nickel-titanium, can still be attached to the medical device to provide increased radiopacity without the need for the material to be directly welded onto the device. The outer layer 44 can be made from a material that can be more easily welded to the marker holder 22 and eliminates possible galvanic corrosion. The temperature of the weld should be set to melt only the portion of the outer layer 44 and the marker holder 34 and not the core 42. It should be appreciated that other materials having high radiopacity can be utilized as the core material and that the outer layer can be made from materials which can be easily welded within the marker holder 22. Additionally, although the radiopaque marker 20 is shown as a substantially rectangular shaped component, it should be appreciated that other shapes and sizes of the radiopaque marker, which includes the encapsulated or partially encapsulated core material, could be utilized. The outer layer 44 can be placed over the inner core 42 utilizing a number of different manufacturing techniques well known in the art. For example, the outer layer could be sputter-coated onto the inner core or formed utilizing extrusion techniques known in the art.

Referring now to FIGS. 6 and 7, yet another marker holder 34 is shown. In the arrangement of FIG. 6, the marker holder 34 has an opening 38 substantially in an hourglass shape. The radiopaque marker 36 is not shown placed within this hourglass shaped opening of FIG. 6, but rather is shown placed within the opening 38 in FIG. 7. In this particular arrangement the marker holder can be made from a nickel-titanium alloy which utilizes the shape memory effect (SME) to hold the radiopaque marker in place. A shape-memory alloy (SMA) utilizes unique properties which allows the material to assume different shapes at different temperatures. Nickel-titanium alloy is a suitable SMA which could be utilized to create the marker holder shown in FIG. 6.

The shape memory effect allows a nickel-titanium alloy to be deformed to a first configuration and then heated or cooled so that the structure returns to another set shape. Nickel-titanium alloys having shape memory effect generally have at least two phases: a martensitic phase, which has a relatively low tensile strength and which is stable at relatively low temperatures, and an austenitic phase, which has a relatively high tensile strength and which is stable at temperatures higher than the martensitic phase.

Shape memory effect is imparted to the alloy by heating the nickel titanium metal to a temperature above which the transformation from the martensitic phase to the austenitic phase is complete; i.e., a temperature above which the austenitic phase is stable. The shape of the metal during this heat treatment is the shape "remembered." The heat treated metal is cooled to a temperature at which the martensitic phase is stable, causing the austenitic phase to transform to the martensitic phase. The metal in the martensitic phase is then plastically deformed, e.g., to facilitate the entry thereof into a patient's body. Subsequent heating of the deformed martensitic phase to a temperature above the martensite to austenite transformation temperature causes the deformed martensitic phase to transform to the austenitic phase. During this phase transformation the metal reverts back towards its original shape.

In this regard, by cooling the stent and marker holder below the martensitic phase temperature, the hourglass shaped opening 38 shown in FIG. 6 can be easily deformed to allow the radiopaque marker 36 to be locked into place, as is shown in FIG. 7. When the temperature of the stent is increased, the shape memory effect creates a tightening force on the radiopaque marker 36 to virtually lock it in place within the marker holder, as is shown in FIG. 7. Alternatively, the superelastic property of nickel-titanium could also be utilized in a similar fashion to tightly fit a suitable radiopaque marker into an opening formed on the marker holder to keep it tightly in place during usage. Alternatively, heat welds or conventional welds could be utilized securely attach the marker to the marker holder. Other suitable bonding techniques could also be utilized to ensure the attachment between these two components, as well.

Shape memory alloys are well known and include, but are not limited to, nickel-titanium and nickel/titanium/vanadium. Any of the shape memory alloys can be formed into a tube and laser cut in order to form the pattern of the stent and marker holder. As is well known, the shape memory alloys of the stent can include the type known as thermoelastic martensitic transformation, or display stress-induced martensite. These types of alloys are well known in the art and need not be further described here.

Importantly, a stent formed of shape memory alloys, whether the thermoelastic or the stress-induced martensite-type, can be delivered using a balloon catheter, or in the case of stress induced martensite, be delivered via a catheter which uses a retractable sheath to restrain the device in a collapsed position. Such catheter delivery systems are well known in the art.

In FIG. 1, it should be remembered that the stent is depicted flat, in a plan view for ease of illustration; however, in use the stent is cylindrically shaped and is generally formed from tubing by laser cutting as described below.

One important feature of a stent made in accordance with the present invention is the capability of the stent to expand from a low-profile diameter to a larger diameter, while still maintaining structural integrity in the expanded state and remaining highly flexible. Stents should usually have an overall expansion ratio of about 1.0 up to about 5.0 times the original diameter, or more, using certain compositions of materials. The stents still retain structural integrity in the expanded state and will serve to hold open the vessel in which they are implanted. Some materials may afford higher or lower expansion ratios without sacrificing structural integrity.

The implantable medical devices of the present invention can be made in many ways. However, one preferred method of making a stent is to cut a thin-walled tubular member, such as Nitinol tubing or stainless steel tubing, to remove portions of the tubing in the desired pattern for the stent, leaving relatively untouched the portions of the metallic tubing which are to form the stent. It is preferred to cut the tubing in the desired pattern by means of a machine-controlled laser.

A suitable composition of Nitinol used in the manufacture of a self-expanding stent of the present invention is approximately 55% nickel and 44.5% titanium (by weight) with trace amounts of other elements making up about 0.5% of the composition. The upper plateau strength is about a minimum of 4.14 x 10⁸Pa (60,000 psi) with an ultimate tensile strength of a minimum of about 1.07 x 10⁹Pa (155,000 psi). The permanent set (after applying 8% strain and unloading), is approximately 0.5%. The breaking elongation is usually a minimum of 10%. It should be appreciated that other compositions of Nitinol can be utilized, as can other self-expanding alloys, to obtain the same features of a self-expanding stent made in accordance with the present invention.

The self-expanding stent of the present invention can be laser cut from a tube of superelastic (sometimes called pseudo-elastic) nickel titanium (Nitinol). All of the stent diameters can be cut with the same stent pattern, and the stent is expanded and heat treated to be stable at the desired final diameter. The heat treatment also controls the transformation temperature of the Nitinol such that the stent is super elastic at body temperature. The transformation temperature is at or below body temperature so that the stent will be superelastic at body temperature. The stent can be electro polished to obtain a smooth finish with a thin layer of titanium oxide placed on the surface. The stent is usually implanted into the target vessel which is smaller than the stent diameter so that the stent applies a force to the vessel wall to keep it open.

The stent tubing of a self-expanding stent made in accordance with the present invention may be made of suitable biocompatible material besides nickel-titanium (NiTi) alloys. In this case the stent would be formed full size but deformed (e.g. compressed) to a smaller diameter onto the balloon of the delivery catheter to facilitate intra luminal delivery to a desired intra luminal site. The stress induced by the deformation transforms the stent from an austenite phase to a martensite phase, and upon release of the force when the stent reaches the desired intra luminal location, allows the stent to expand due to the transformation back to the more stable austenite phase.

The medical device also may be made of suitable biocompatible material such as stainless steel. The stainless steel may be alloy-type: 316L SS, Special Chemistry per ASTM F138-92 or ASTM F139-92 grade 2.

While it is preferred that stents be made in accordance with the present invention be fabricated from laser cut tubing, those skilled in the art will realize that the stent can be laser cut from a flat sheet and then rolled up in a cylindrical configuration with the longitudinal edges welded to form a cylindrical member. Other methods of forming the stent of the present invention can be used, such as chemical etching; electric discharge machining; laser cutting a flat sheet and rolling it into a cylinder; and the like, all of which are well known in the art at this time.

Due to the thin wall and the small geometry of the stent pattern, it is necessary to have very precise control of the laser, its power level, the focus spot size, and the precise positioning of the laser cutting path. In cutting the strut widths and marker holders, it is preferable to have a very focused laser spot size which will allow the precise strut pattern to be created on the tubing. For this reason, additional instrumentation which includes a series of lenses may be necessary to be utilized with the laser in order to create the fine focused laser spot necessary to cut that particular pattern.

Generally, the tubing is put in a rotatable collet fixture of a machine-controlled apparatus for positioning the tubing relative to a laser. According to machine-encoded instructions, the tubing is then rotated and moved longitudinally relative to the laser which is also machine-controlled. The laser selectively removes the material from the tubing by ablation and a pattern is cut into the tube. The tube is therefore cut into the discrete pattern of the finished stent, which includes the desired number of marker holders. Further details on how the tubing can be cut by a laser are found in U.S. Patent Nos. 5,759,192 (Saunders) and 5,780,807 (Saunders).

The process of cutting a pattern for the stent into the tubing generally is automated except for loading and unloading the length of tubing. For example, a pattern can be cut in tubing using a CNC-opposing collet fixture for axial rotation of the length of tubing, in conjunction with CNC X/Y table to move the length of tubing axially relative to a machine-controlled laser as described. The entire space between collets can be patterned using the CO₂ or Nd:YAG laser set-up. The program for control of the apparatus is dependent on the particular configuration used and the pattern to be ablated in the coding.

After the stent has been cut by the laser, electrical chemical polishing, using various techniques known in the art, should be employed in order to create the desired final polished finish for the stent. The electropolishing will also be able to take off protruding edges and rough surfaces which were created during the laser cutting procedure.

While the invention has been illustrated and described herein, in terms of its use as a stent, it will be apparent to those skilled in the art that the medical device can take on a number of different forms and a number of different applications. Other modifications and improvements may be made without departing from the scope of the invention.

## Claims

1. An implantable medical device having enhanced radiopacity, comprising:
a structural body formed from a biocompatible material having a certain level of radiopacity, the structural body including at least one marker holder (22) integrally formed therein; and
a radiopaque marker (20) made from a material having a level of radiopacity greater than the level of radiopacity of the biocompatible material from which the structural body is formed, the radiopaque marker (20) being attachable within the marker holder (22), wherein the marker holder (22) includes a pair of projecting fingers (26) which define an opening (30) and the radiopaque marker (20) includes a region (32) which fits within the opening (30) defined by the projecting fingers (26), **characterised in that** the opening (30) defined by the projecting fingers (26) of the marker holder (22) is V-shaped and the region (32) formed on the radiopaque marker (20) to fit within the opening (30) is V-shaped.

2. The implantable medical device of claim 1, wherein the radiopaque marker (20) is attached to the projecting fingers (26) of the marker holder (22) by a heat weld.

3. The implantable medical device of claim 1, wherein the projecting fingers (26) are connected at a notched region (28) which allows the projecting fingers (26) to move laterally to accept the radiopaque marker (20).

4. The implantable medical device of claim 3, wherein the radiopaque marker (20) has a region (32) which fits within the opening (30) defined by the projecting fingers (26) of the marker holder (22) and the radiopaque marker (20) and projecting fingers (26) are bonded together by a heat weld.

5. The implantable medical device of claim 1, wherein the V-shaped opening (30) defined by the projecting fingers (26) defines a particular angle (α) and the V-shaped region (32) of the radiopaque marker (20) defines an angle (β) which is larger than the angle (α) of the V-shaped opening (30).

6. The implantable medical device of claim 3, wherein the radiopaque marker (20) has a region (32) adapted to fit within the opening (30) defined by the projecting fingers (26) that is larger than the opening (30) defined by the projecting fingers (26).

7. The implantable medical device of claim 1, wherein the structural body is formed from a superelastic alloy having a certain level of radiopacity, and the radiopaque marker (20) is made from a nickel-titanium alloy including a ternary element which attains a level of radiopacity greater than the level of radiopacity of the superelastic alloy from which the structural body is formed.

8. The implantable medical device of claim 1, wherein the radiopaque marker (20) includes a core at least partially encapsulated by an outer layer, the material forming the core having a level of radiopacity greater than the level of radiopacity of the material forming the structural body, the radiopaque marker (20) being adapted to fit within the marker holder (22).

## Patentansprüche

1. Implantierbare medizinische Vorrichtung mit verbesserter Strahlenundurchlässigkeit umfassend:
einen Strukturkörper ausgebildet aus einem biokompatiblen Material mit einem bestimmten Grad an Strahlenundurchlässigkeit, wobei der Strukturkörper wenigstens eine Markerhaltevorrichtung (22) umfasst, welche darin integral ausgebildet ist, und
einen strahlenundurchlässigen Marker (20), welcher hergestellt ist aus einem Material mit einem Grad an Strahlenundurchlässigkeit der größer ist als der Grad an Strahlenundurchlässigkeit des biokompatiblen Materials, aus welchem der Strukturkörper ausgebildet ist, wobei der strahlenundurchlässige Marker (20) innerhalb der Markerhaltevorrichtung (22) anbringbar ist, wobei die Markerhaltevorrichtung (22) ein Paar an vorstehenden Fingern (26) umfasst, welche eine Öffnung (30) definieren und der strahlenundurchlässige Marker (20) einen Bereich (32) umfasst, welcher in die Öffnung (30) passt, welche definiert ist durch die vorstehenden Finger (26),
**dadurch gekennzeichnet, dass** die Öffnung (30), welche durch die vorstehenden Finger (26) der Markerhaltevorrichtung (22) definiert ist, V-förmig ist und dass der Bereich (32), welcher auf dem strahlenundurchlässigen Marker (20) so ausgebildet ist, dass er die Öffnung (30) passt, V-förmig ist.

2. Die implantierbare medizinische Vorrichtung nach Anspruch 1, wobei der strahlenundurchlässige Marker (20) an die vorstehenden Finger (26) auf der Markerhaltevorrichtung (22) mittels Wärmeschweißen befestigt ist.

3. Die implantierbare medizinische Vorrichtung nach Anspruch 1, wobei die vorstehenden Finger (26) mit einem gekerbten Bereich (28) verbunden sind, welcher es den vorstehenden Fingern (26) ermöglicht, sich seitlich zu bewegen, um den strahlenundurchlässigen Marker (20) aufzunehmen.

4. Die implantierbare medizinische Vorrichtung nach Anspruch 3, wobei der strahlenundurchlässige Marker (20) einen Bereich (32) aufweist, welcher in die Öffnung (30) passt, welche definiert ist durch die vorstehenden Finger (26) der Markerhaltevorrichtung (22), und wobei der strahlenundurchlässige Marker (20) und die vorstehenden Finger (26) mittels Wärmeschweißen aneinander gebunden sind.

5. Die implantierbare medizinische Vorrichtung nach Anspruch 1, wobei die V-förmige Öffnung (30), welche durch die vorstehenden Finger (26) definiert ist, einen bestimmten Winkel (α) definiert und der V-förmige Bereich (32) des strahlenundurchlässigen Markers (20) einen Winkel (β) definiert, welcher größer ist als der Winkel (α) der V-förmigen Öffnung (30).

6. Die implantierbare medizinische Vorrichtung nach Anspruch 3, wobei der strahlenundurchlässige Marker (20) einen Bereich (32) aufweist, welcher so angepasst ist, dass er in die Öffnung (30) passt, welche definiert ist durch die vorstehenden Finger (26), welcher größer ist als die durch die vorstehenden Finger (26) Öffnung (30).

7. Die implantierbare medizinische Vorrichtung nach Anspruch 1, wobei der Strukturkörper ausgebildet ist aus einer superelastischen Legierung mit einem bestimmten Grad an Strahlenundurchlässigkeit und der strahlenundurchlässige Marker (20) hergestellt ist aus einer Nickel-Titan-Legierung umfassend ein ternäres Element, welches einen Grad an Strahlenundurchlässigkeit erzielt, welcher größer ist als der Grad an Strahlenundurchlässigkeit der superelastischen Legierung, aus welcher der Strukturkörper ausgebildet ist.

8. Die implantierbare medizinische Vorrichtung nach Anspruch 1, wobei der strahlenundurchlässige Marker (20) einen Kern umfasst, welcher wenigstens teilweise durch eine äußere Schicht verkapselt ist, wobei das Material, welches den Kern ausbildet, einen Grad an Strahlenundurchlässigkeit aufweist, welcher größer ist als der Grad an Strahlenundurchlässigkeit des Materials, welches den Strukturkörper ausbildet, wobei der strahlenundurchlässige Marker (20) so angepasst ist, dass er in die Markerhaltevorrichtung (22) passt.

## Revendications

1. Dispositif médical implantable ayant une radio-opacité renforcée, comportant :
un corps structurel formé d'une matière biocompatible ayant un certain niveau de radio-opacité, le corps structurel comprenant au moins un support (22) de marqueur formé de façon intégrée en lui ; et
un marqueur radio-opaque (20) formé d'une matière ayant un niveau de radio-opacité supérieur au niveau de radio-opacité de la matière biocompatible dont est formé le corps structurel, le marqueur radio-opaque (20) pouvant être attaché dans le support (22) de marqueur, dans lequel le support (22) de marqueur comprend une paire de doigts (26) en saillie qui définissent une ouverture (30) et le marqueur radio-opaque (20) comprend une région (32) qui s'ajuste dans l'ouverture (30) définie par les doigts (26) en saillie, **caractérisé en ce que** l'ouverture (30) définie par les doigts (26) en saillie du support (22) de marqueur est en forme de V et la région (32) formée sur le marqueur radio-opaque (20) pour s'ajuster dans l'ouverture (30) est en forme de V.

2. Dispositif médical implantable selon la revendication 1, dans lequel le marqueur radio-opaque (20) est attaché aux doigts en saillie (26) du support (22) de marqueur par une soudure à chaud.

3. Dispositif médical implantable selon la revendication 1, dans lequel les doigts en saillie (26) sont reliés au niveau d'une région échancrée (28) qui permet aux doigts en saillie (26) de se déplacer latéralement pour recevoir le marqueur radio-opaque (20).

4. Dispositif médical implantable selon la revendication 3, dans lequel le marqueur radio-opaque (20) comporte une région (32) qui s'ajuste dans l'ouverture (30) définie par les doigts (26) en saillie du support (22) de marqueur et le marqueur radio-opaque (20) et les doigts (26) en saillie sont liés entre eux par une soudure à chaud.

5. Dispositif médical implantable selon la revendication 1, dans lequel l'ouverture (30) en forme de V définie par les doigts (26) en saillie définit un angle particulier (α) et la région (32) en forme de V du marqueur radio-opaque (20) définit un angle (β) qui est supérieur à l'angle (α) de l'ouverture (30) en forme de V.

6. Dispositif médical implantable selon la revendication 3, dans lequel le marqueur radio-opaque (20) comporte une région (32) conçue pour s'ajuster dans l'ouverture (30) définie par les doigts (26) en saillie, qui est plus grande que l'ouverture (30) définie par les doigts (26) en saillie.

7. Dispositif médical implantable selon la revendication 1, dans lequel le corps structurel est formé d'un alliage superélastique ayant un certain niveau de radio-opacité, et le marqueur radio-opaque (20) est formé d'un alliage nickel-titane comprenant un élément ternaire qui atteint un niveau de radio-opacité supérieur au niveau de radio-opacité de l'alliage superélastique dont est formé le corps structurel.

8. Dispositif médical implantable selon la revendication 1, dans lequel le marqueur radio-opaque (20) comprend un noyau au moins partiellement encapsulé par une couche extérieure, la matière formant le noyau ayant un niveau de radio-opacité supérieur au niveau de radio-opacité de la matière formant le corps structurel, le marqueur radio-opaque (20) étant conçu pour s'ajuster dans le support (22) de marqueur.
